# EUROPEAN PATENT APPLICATION

(11) **EP 4 520 801 A1**
(43) Date of publication of application: **12.03.2025**
(21) Application number: 23196187.1
(22) Date of filing: 08.09.2023
(51) Int. Cl.: C09K 3/22, C12N 1/20

(54) **DUST SUPPRESSANT SOLID FORMULATION**

(71) Applicant: Earth Alive Clean Technologies Inc., Montréal, Québec H2Z 1S4 (CA)
(72) Inventor: TOUSSAINT, Claudia, Montréal, H2Z 1S4 (CA); NEUFELD, Simon, Montréal, H2Z 1S4 (CA); SAFINA, Gulnaz, Montréal, H2Z 1S4 (CA)
(74) Representative: Calysta NV

(57) **Abstract**

Dust suppressant solid formulation to be diluted in an aqueous phase, comprising a first microbial fraction comprising a series of biofilm forming bacteria species and a second non-microbial fraction, comprising at least latex binder.

## Description

The present invention relates to a dust suppressant solid formulation to be diluted in an aqueous phase.

Dust suppressant solutions are commonly used in mining roads, heavy-traffic roads, unpaved public roads and other industrial operations to control the generation and spread of dust on roads caused by haul trucks and other heavy equipment and the dispersion of fugitive dust on stockpiles. These solutions are typically chemical compounds that are applied to the road or stockpile surface to minimize the release of dust particles into the air. The use of dust suppressants helps improve air quality, reduce health risks, increase safety for mine workers, and minimize environmental impacts.

There are several types of dust suppressants available, including water-based solutions, petroleum-based products, synthetic compounds, and biological solutions. Each type has its own advantages, disadvantages and considerations in terms of effectiveness, cost, and environmental impact.

Water-based dust suppressants are widely used due to their availability and low cost. They are also called humidifying agents. These solutions consist primarily of water and may contain additional additives, like salts and chloride and/or electrochemical products, to enhance their dust control properties. When applied to the road surface, water suppresses dust by weighing down the particles and reducing their ability to become airborne. Water-based solutions are typically applied through sprinklers, water trucks, or water cannons. While they are effective in temporarily reducing dust, their effectiveness may be limited in areas with low precipitation or high traffic volume. Frequent reapplication may be required to maintain dust control. The use of salt and chloride may also lead to increased corrosion of the machinery and to leaching into the environment.

Petroleum-based dust suppressants utilize hydrocarbon-based compounds to control dust on mine roads. These products are often derived from crude oil or its byproducts. Petroleum-based solutions work by binding dust particles together, forming a film on the road surface, or reducing the surface tension of the particles. These compounds have good adhesive properties, providing longer-lasting dust control compared to water-based solutions. However, they may have higher costs and potential environmental concerns due to their petroleum-derived nature. In addition, they have a tendency to crack with humidity. Careful consideration of environmental impacts and adherence to regulatory guidelines are crucial when using petroleum-based products.

Synthetic dust suppressant compounds utilize chemistry and synthetic compounds to control dust on mine roads. These products are generally non-corrosive for the machinery and are relatively easy to be applied in various locations. These artificial and synthetic products can reduce up to 35% of the dust. However, these products depend strongly on soil characteristics and presence of specific minerals and the performance are unpredictable.

Regarding biological dust suppressant formulations, this product is known in the art, see for example document WO2014/198840. That document discloses a dust suppressant composition comprising a carrier and microorganisms with a vague disclosure on the physical state of the compositions, but a careful reading reveals that only liquid formulations are described.

In that document, Example 1 composition comprises more than 95% of liquids (water and glycerin). Example 2 uses approximately the same composition as example 1. Example 3 explicitly states that it comprises a liquid carrier. Example 4 evaluates the dust suppressing effect in a different liquid carrier. Examples 5 and 6 use the same dust suppressant liquid composition as example 1. Example 7 uses a solution of TY broth at a concentration of 10% v/v as carrier.

The dust suppressant effect of the liquid compositions of document WO2014/198840 involves two distinct periods of activity, dependent on when the liquid composition is applied. The liquid compositions disclosed typically consist of two compounds, namely glycerin and water. The first period of activity starts immediately after applying the liquid compositions. In this first period of activity, water and glycerin play a role in dust suppression by moistening the soil and preventing dust from becoming airborne. The second period of activity begins when the bacteria within the liquid composition proliferate and generate a biofilm. This biofilm solidifies, effectively binding the dust particles together, thereby enhancing the dust suppressant effect.

Unfortunately, with such liquid compositions whether they are water-based or liquid biological, large volumes are required, which means high energy and cost requirements for transporting and storing them.

In the context of environmental considerations, it is essential to carefully evaluate the impact of dust suppressant solutions on the surrounding ecosystem. While controlling dust is crucial for maintaining air quality and safeguarding the health of workers, it is equally important to minimize any potential adverse effect on the environment.

Liquid formulations are perceived as inappropriate due to the implications of water storage and transportation required to reach their final destination, which could be considered environmentally detrimental.

The dissolution of bacteria in an aqueous phase presents a challenging problem due to their inherent hydrophobic nature. Bacterial cell membranes are composed of lipids and proteins that repel water, making it difficult for them to disperse and dissolve uniformly in an aqueous medium.

The present invention endeavors to solve at least a part of these drawbacks by providing a multifunctional solid concentrate that can be used to prepare liquid formulations directly where it has to be applied, or can be used to enhance the properties of other compositions.

Another problem to be solved by the present invention is to be able to provide a dust suppressant solid formulation, that can be easily prepared, stored, transported and diluted for use directly at the treatment site, and for which the dust suppressant activity of the bacteria is preserved over time.

To solve these problems, it is provided according to the present invention, a dust suppressant solid formulation to be diluted in an aqueous phase, comprising a first microbial fraction comprising a series of biofilm forming bacteria species and a second non-microbial fraction comprising at least latex binder.

As can be seen, the formulation comprises 2 elements, i.e. a latex binder and a series of biofilm forming bacteria species.

The latex binder is a dispersion of microscopic polymer particles in water. The particles do not sink or float in a water-based emulsion, nor do they coagulate due to ionic or steric instability. Ionic stability is the result of ionic charges on particles, producing a repulsive force that prevents agglomeration.

For example, in paper coating, latex is used to anchor the pigments to the raw paper surface thus giving quality paper the desired finish and excellent printing quality, in the production of floor coverings, latex is used to bond pigments to textile fibers and these fibers to each other.

Surprisingly, it has been shown that the binding properties are also achieved with particles of dust and when applied to a road surface, where they help create a resilient surface that can withstand the stresses of traffic and weather conditions while avoiding airborne particles. This improved condition plays further a role in reducing the braking distance on treated roads. As vehicles traverse the road, the latex-treated surface absorbs and disperses energy, resulting in better traction and grip. Consequently, this leads to shorter braking distances and enhanced overall road safety.

In addition, the environmentally friendly nature of latex makes it a sustainable option, reducing the environmental impact compared to traditional dust suppressant materials for example petroleum-based or synthetic.

Further, said latex is biocompatible with said series of dust suppressing bacteria species. This biocompatible nature allows for a seamless integration with said series of dust suppressing bacteria species.

According to the present invention, said series of dust suppressing bacteria species actively generate a biofilm on the road surface that starts growing quite quickly (within 48 hours) and continues to grow, expand and solidify over time, while the non-microbial fraction together with the aqueous phase act as dust suppressant during the initial period of time (at least during 48h). This biofilm serves as a protective matrix, providing an ideal habitat for the bacteria to thrive and grow. As the dust suppressant is applied, the bacteria initiate the formation of the biofilm, effectively binding themselves to the road surface. The biofilm enhances their ability to adhere firmly, ensuring maximum coverage and prolonged activity.

Further, the dust suppressant solid formulation dissolves readily when mixed with liquids in general, and with liquid dust suppressants specifically.

In addition, said dust suppressant solid formulation is more convenient to store and to transport. It is less prone to spillage and leakage than liquid formulations, making it safer and more straightforward to handle.

Further, using a latex binder to solubilize bacteria in an aqueous phase offers a significant advantage by improving the dispersion of bacteria in liquid. The latex binder acts as an emulsifier, helping to mix the bacteria and the aqueous phase, resulting in a more homogenous solubilization.

Other embodiments of the dust suppressant solid formulation are mentioned in the appended claims.

Preferably, said second non-microbial fraction of the dust suppressant solid formulation of the present invention comprises a further binder chosen from the group comprising lignosulfonate, xanthan gum, acacia gum, cellulose and the like, their derivates and their combination.

Advantageously, the latex binder of the dust suppressant solid formulation of the present invention is a biobased latex binder, in particular a biobased latex binder having surfactant properties, more preferably a biobased starch latex binder, more preferably a biobased modified starch latex binder.

In addition, using a biobased latex binder contributes to a more sustainable and environmentally conscious approach. Biobased latex binders are derived from renewable resources, such as plant material, making them a greener choice that helps reduce the overall carbon footprint.

Further, biobased latex binders demonstrate excellent biocompatibility, ensuring the stability and longevity of the bacteria.

Moreover, the surfactant properties of the biobased latex binder enhance its effectiveness in solubilizing bacteria in aqueous phases. Surfactants are surface-active agents that reduce the surface tension between liquids, enabling better mixing and dispersion of the binder within the aqueous medium. When used for solubilizing bacteria, the surfactant properties of the biobased latex binder promote faster and more efficient emulsification, creating a homogenous mixture with the bacteria. This results in more effective solubilization, saving valuable effort.

Furthermore, the surfactant nature of the biobased latex binder enhances its ability to stabilize the solubilized bacteria over time. For example, by forming micelles or stabilizing the interface between the bacteria and the aqueous phase, it helps prevent aggregation or precipitation, ensuring the integrity of the bacterial solution even during extended storage periods.

In addition, starch latex binders have excellent biodegradability, allowing them to naturally break down over time without leaving harmful residues. This characteristic is beneficial to reduce waste and environmental pollution.

Further, one significant advantage of a biobased modified starch latex binder lies in its enhanced performance compared to traditional starch binders. Through modification, the binder gains improved stability, versatility, and adhesive properties. This results in stronger and more durable bonding.

Preferably, the dust suppressant solid formulation of the present invention comprises from 95 to 99.9 weight% of the second non-microbial fraction with respect to the total weight of the dust suppressant solid formulation and from 0.1 to 5 weight% of the first microbial fraction with respect to the total weight of the dust suppressant solid formulation, preferably comprising from 95 to 99.5 weight% of said second non-microbial fraction and from 0.5 to 5 weight % of said microbial fraction, advantageously comprising from 96 to 99 weight% of said second non-microbial fraction and from 1 to 4 weight% of said first microbial fraction, more preferably comprising from 96 to 98 weight% of said second non-microbial fraction and from 2 to 4 weight% of said first microbial fraction, even more preferably comprising from 97 to 98 weight% of said second non-microbial fraction and from 2 to 3 weight% of said first microbial fraction.

Surprisingly, the inventors found that a ratio of the second non-microbial fraction in proportion from 95% to 99.9% in weight with respect to the total weight of the solid formulation with the series of biofilm forming bacteria species in proportion from 0.1% to 5% in weight is particularly suitable for achieving the technical objectives of the present invention.

Advantageously, said second non-microbial fraction of the dust suppressant solid formulation of the present invention is in the form of a powder.

This offers the advantage of a solid formulation of the present invention in which the series of bacteria is in solid form, as is the second non-microbial fraction, making it easier to transport, store and ensure stability over time.

Preferably, said series of biofilm forming bacteria species of the dust suppressant solid formulation of the present invention comprises one or more biofilm forming bacteria species chosen in the group comprising Achromobacter, Acetonema, Actinobacter, Alcaligenes, Alkalibacillus, Ammoniphilus, Amphibacillus, Anaerobacter, Anaerospora, Aneurinibacillus, Anoxybacillus, Arthrobacter, Bacillus, Brevibacillus, Caldanaerobacter, Caloramator, Caminicella, Cerasibacillus, Clostridium, Clostridiisalibacter, Cohnella, Dendrosporobacter, Desulfotomaculum, Desulfosporomusa, Desulfosporosinus, Desulfovirgula, Desulfunispora, Desulfurispora, Enterobacter, Filifactor, Filobacillus, Flavobacterium, Gelria, Geobacillus, Geosporobacter, Gracilibacillus, Halonatronum, Heliobacterium, Heliophilum, Laceyella, Lentibacillus, Lysinibacillus, Mahella, Metabacterium, Moorella, Natroniella, Oceanobacillus, Orenia, Ornithinibacillus, Oxalophagus, Oxobacter, Paenibacillus, Paraliobacillus, Pelospora, Pelotomaculum, Piscibacillus, Planifilum, Pontibacillus, Pseudomonas, Propionispora, Rhodococcus, Salinibacillus, Salsuginibacillus, Seinonella, Shimazuella, Sporacetigenium, Sporoanaerobacter, Sporobacter, Sporobacterium, Sporohalobacter, Sporolactobacillus, Sporomusa, Sporosarcina, Sporotalea, Sporotomaculum, Syntrophomonas, Syntrophospora, Tenuibacillus, Tepidibacter, Terribacillus, Thalassobacillus, Thermoacetogenium, Thermoactinomyces, Thermoalkalibacillus, Thermoanaerobacter, Thermoanaeromonas, Thermobacillus, Thermoflavimicrobium, Thermovenabulum, Tuberibacillus, Virgibacillus, Vulcanobacillus, preferably comprising two or more biofilm forming bacteria species, more preferably comprising three or more biofilm forming bacteria species, more preferably comprising four or more biofilm forming bacteria species, even more preferably comprising five or more biofilm forming bacteria species.

Advantageously, said series of biofilm forming bacteria species of the dust suppressant solid formulation of the present invention comprises one or more biofilm forming bacteria species chosen in the group comprising Bacillus amyloliquefaciens, Bacillus licheniformis, Bacillus megaterium, Bacillus pumilis, Bacillus subtilis, Bacillus subtilis subspecies Inaquosorum, Bacillus subtilis subspecies subtilis, Bacillus velezensis, preferably comprising two or more biofilm forming bacteria species, more preferably comprising three or more biofilm forming bacteria species, more preferably comprising four or more biofilm forming bacteria species, even more preferably comprising five or more biofilm forming bacteria species.

Preferably, the series of biofilm forming bacteria species of the dust suppressant solid formulation of the present invention contains at least 500 million (5×10⁸) cfu total biofilm forming bacteria per gram of said dust suppressant solid formulation, preferably contains at least 600 million (6×10⁸) cfu total biofilm forming bacteria per gram of said dust suppressant solid formulation, more preferably contains at least 700 million (7×10⁸) cfu total biofilm forming bacteria per gram of said dust suppressant solid formulation, even more preferably contains at least 800 million (8×10⁸) cfu total biofilm forming bacteria per gram of said dust suppressant solid formulation, advantageously contains at least 900 million (9×10⁸) cfu total biofilm forming bacteria per gram of said dust suppressant solid formulation, more advantageously contains at least 1 billion (1×10⁹) cfu total biofilm forming bacteria per gram of said dust suppressant solid formulation, preferably contains at least 1.5 billion (1.5×10⁹) cfu total biofilm forming bacteria per gram of said dust suppressant solid formulation, more preferably contains at least 2 billion (2×10⁹) cfu total biofilm forming bacteria per gram of said dust suppressant solid formulation, even more preferably contains at least 2.5 billion (2.5×10⁹) cfu total biofilm forming bacteria per gram of said dust suppressant solid formulation, preferably contains at least 3 billion (3×10⁹) cfu total biofilm forming bacteria per gram of said dust suppressant solid formulation, more preferably contains at least 3.5 billion (3.5×10⁹) cfu total biofilm forming bacteria per gram of said dust suppressant solid formulation, even more preferably contains at least 4 billion (4×10⁹) cfu total biofilm forming bacteria per gram of said dust suppressant solid formulation, advantageously contains at least 4.5 billion (4.5×10⁹) cfu total biofilm forming bacteria per gram of said dust suppressant solid formulation, even more preferably contains at least 5 billion (5×10⁹) cfu total biofilm forming bacteria per gram of said dust suppressant solid formulation, in particular contains at least 5.5 billion (5.5×10⁹) cfu total biofilm forming bacteria per gram of said dust suppressant solid formulation, preferably contains at least 6 billion (6×10⁹) cfu total biofilm forming bacteria per gram of said dust suppressant solid formulation, more preferably contains at least 6.5 billion (6.5×10⁹) cfu total biofilm forming bacteria per gram of said dust suppressant solid formulation, in particular contains at least 7 billion (7×10⁹) cfu total biofilm forming bacteria per gram of said dust suppressant solid formulation, more particular contains at least 7.5 billion (7.5×10⁹) cfu total biofilm forming bacteria per gram of said dust suppressant solid formulation, even more preferably contains at least 8 billion (8×10⁹) cfu total biofilm forming bacteria per gram of said dust suppressant solid formulation, preferably contains at least 9 billion (9×10⁹) cfu total biofilm forming bacteria per gram of said dust suppressant solid formulation, advantageously contains at least 10 billion (1×10¹⁰) cfu total biofilm forming bacteria per gram of said dust suppressant solid formulation.

Preferably, said series of biofilm forming bacteria species of the dust suppressant solid formulation of the present invention contains:
- at least 0.1 million (1×10⁵), in particular at least 0.5 million (5×10⁵), more particular at least 0.8 million (8×10⁵), preferably at least 1 million (1×10⁶), more preferably at least 2 million (2×10⁶), in particular at least 4 million (4×10⁶), particularly at least 4.7 million (4.7×10⁶), preferably at least 5 million (5×10⁶), more preferably at least 10 million (1×10⁷), in particular at least 20 million (2×10⁷), particularly at least 50 million (5×10⁷) preferably at least 70 million (7×10⁷), more preferably at least 100 million (1×10⁸), particularly at least 150 million (1.5×10⁸) preferably at least 200 million (2×10⁸), more preferably at least 250 million (2.5×10⁸), in particular at least 300 million (3×10⁸), particularly at least 350 million (3.5×10⁸), more preferably at least 400 million (4×10⁸), particularly at least 450 million (4.5×10⁸), more preferably at least 500 million (5×10⁸), cfu of Bacillus licheniformis per gram of the dust suppressant solid formulation measured according to ASTM-D5465-93 (dated 1998) standard, and/or
- at least 10 million (1×10⁷), preferably at least 50 million (5×10⁷), more preferably at least 100 million (1×10⁸), in particular at least 200 million (2×10⁸), particularly at least 300 million (3×10⁸), advantageously at least 400 million (4×10⁸), more particular at least 500 million (6×10⁸), in particular at least 700 million (7×10⁸), preferably at least 800 million (8×10⁸), even more preferably at least 900 million (9×10⁸), for example at least 920 million (9.2×10⁸), preferably at least 950 million (9.5×10⁸), more preferably at least 1 billion (1×10⁹), cfu of Bacillus megaterium per gram of the dust suppressant solid formulation measured according to ASTM-D5465-93 (dated 1998) standard, and/or
- at least 100 thousand (1×10⁵), preferably at least 300 thousand (3×10⁵), more particular 500 thousand (5×10⁵), more preferably at least 830 thousand (8.3×10⁵), in particular at least 1 million (1×10⁶), particularly at least 2 million (2×10⁶), advantageously at least 3 million (3×10⁶), more particular at least 4 million (4×10⁶), cfu of Bacillus amyloliquefaciens per gram of the dust suppressant solid formulation measured according to ASTM-D5465-93 (dated 1998) standard, and/or
- at least 100 million (1×10⁸), particularly at least 250 million (2.5×10⁸) preferably at least 400 million (4×10⁸), more preferably at least 600 million (6×10⁸), in particular at least 750 million (7.5×10⁸), particularly at least 800 million (8×10⁸), more preferably at least 900 million (9×10⁸), in particular at least 1 billion (1×10⁹), preferably at least 2 billion (2×10⁹), more particular 2.8 billion (2.8×10⁹), more preferably at least 3.6 billion (3.6×10⁹), in particular at least 5 billion (5×10⁹), particularly at least 7 billion (7×10⁹), advantageously at least 7.2 billion (7.2×10⁹), more particular at least 8 billion (8×10⁹), particularly at least 8.5 billion (8.5×10⁹), cfu of Bacillus subtilis per gram of the dust suppressant solid formulation measured according to ASTM-D5465-93 (dated 1998) standard, and/or
- at least 0.1 billion (1×10⁸), preferably at least 0.3 billion (3×10⁸), more particular 0.5 billion (5×10⁸), more preferably at least 0.8 billion (8×10⁸), in particular at least 1 billion (1×10⁹), particularly at least 2 billion (2×10⁹), advantageously at least 3 billion (3×10⁹), more particular at least 3.2 billion (3.2x10⁹), preferably at least 4 billion (4×10⁹), advantageously at least 5 billion (5×10⁹), more particular at least 7 billion (7×10⁹) cfu of Bacillus subtilis subspecies Inaquosorum per gram of the dust suppressant solid formulation measured according to ASTM-D5465-93 (dated 1998) standard, and/or
- at least 0.1 billion (1×10⁸), preferably at least 0.3 billion (3×10⁸), more particular 0.5 billion (5×10⁸), more preferably at least 0.8 billion (8×10⁸), in particular at least 1 billion (1×10⁹), particularly at least 2 billion (2×10⁹), advantageously at least 3 billion (3×10⁹), more particular at least 3.2 billion (3.2x10⁹), preferably at least 4 billion (4×10⁹), advantageously at least 5 billion (5×10⁹), more particular at least 7 billion (7×10⁹) cfu of Bacillus subtilis subspecies subtilis per gram of the dust suppressant solid formulation measured according to ASTM-D5465-93 (dated 1998) standard, and/or
- at least 10 million (1×10⁷), preferably at least 50 million (5×10⁷), more particular 100 million (1×10⁸), more preferably at least 200 million (2×10⁸), in particular at least 300 million (3×10⁸), particularly at least 350 million (3.5×10⁸), advantageously at least 400 million (4×10⁸), more particular at least 450 million (4.5×10⁸), even more preferably at least 500 million (5×10⁸) cfu of Bacillus pumilis per gram of the dust suppressant solid formulation measured according to ASTM-D5465-93 (dated 1998) standard, and/or
- at least 0.1 billion (1×10⁸), preferably at least 0.5 billion (5×10⁸), more particular 0.8 billion (8×10⁸), more preferably at least 1 billion (1×10⁹), in particular at least 2 billion (2×10⁹), particularly at least 2.5 billion (2.5×10⁹), advantageously at least 3 billion (3×10⁹), more particular at least 3.5 billion (3.5×10⁹), even more preferably at least 4 billion (4×10⁹), advantageously at least 4.5 billion (4.5×10⁹), in particular at least 5 billion (5×10⁹) cfu of Bacillus velezensis per gram of the dust suppressant solid formulation measured according to ASTM-D5465-93 (dated 1998) standard.

Advantageously, the second non-microbial fraction of the dust suppressant solid formulation of the invention comprises one or more solid additives chosen from the group comprising humectants, viscosity modifiers, preservatives, surfactants, dispersants, electrolytes, emulsifying agents, pH modifiers, pigments, deicers, and their combinations thereof.

Preferably, said humectant is chosen in the group comprising lignosulfonates, glycerin, sugar alcohols for example sorbitol, xylitol, molasses, vegetable oils and their combinations thereof.

Preferably, said preservative is 2-bromo-2-nitropropane-1,3-diol (bronopol) and the like.

Preferably, said surfactant is chosen in the group of Lutensol^{®} line of surfactants, Yucca schidigera extracts, ethoxylated alkyl phenols, organomodified silicones, rhamnolipids, sophorolipids, alcohol ethoxylates, and their combinations thereof.

Preferably, said deicer is an eco-friendly deicer, for example potassium formate and the like.

Preferably, the one or more additive of said dust suppressant solid formulation of the present invention is in solid form.

The present invention also relates to a dust suppressant liquid composition comprising:
- from 10 to 99 weight% of water based on the weight of said liquid composition,
- from 10 to 90 weight% of one or more additive based on the weight of said liquid composition,
- from 0.1 to 30 weight% of the dust suppressant solid formulation according to the invention based on the weight of said liquid composition.

The dust suppressant liquid composition of the present invention advantageously presents a triple action when applied to the substrate to be treated. Firstly, the water and the one or more additives of said liquid composition will act immediately as humidifying agent by weighing down the dust particles and reducing their ability to become airborne. Secondly and in parallel, the latex binder together with the aqueous phase will act both as creating a flexible and resilient surface avoiding airborne particles and as protective matrix for the growing of the bacteria to initiate the formation of the biofilm. Thirdly and in parallel, due to the presence of the latex binder as a food source and carbon supply for said series of dust suppressing bacteria species, the bacteria actively generate a biofilm on the road surface enhancing their ability to adhere firmly and ensuring maximum coverage and prolonged activity to suppress dust emissions.

Other embodiments of the dust suppressant liquid composition according to the present invention are mentioned in the appended claims.

Advantageously, said dust suppressant liquid composition of the present invention comprises:
- from 15 w% to 99 w%, in particular from 20 w% to 99w%, more particular from 25 w% to 99 w%, even more particular 30 w% to 99 w%, advantageously from 35 w% to 99w% preferably from 40 w% to 99 w%, more preferably from 50 w% to 99 w%, even more preferably from 60 w% to 99 w%, advantageously from 70 w% to 99 w%, in particular from 75 w% to 99 w% of water based on the weight of said liquid composition, and/or
- from 10 w% to 85 w%, preferably from 10 w% to 80 w%, more preferably from 10 w% to 70 w%, even more preferably from 10 w% to 60 w%, advantageously from 10 w% to 50 w%, in particular from 10 w% to 40 w%, particularly from 10 w% to 30 w%, even more preferably from 10 w% to 25 w%, advantageously from 15 w% to 20 w% of said one or more additive based on the weight of said liquid composition, and/or
- from 0.1 w% to 30 w% of the dust suppressant solid formulation according to the invention based on the weight of said liquid composition, for example 0.5 w%, 1 w%, 1.5 w%, 2.0 w%, 3.0 w%, 4.0 w%, 5.0 w%, 6.0 w%, 7.0 w%, 8.0 w%, 9.0 w%, 10.0 w%, 11.0 w%, 12.0 w%, 13.0 w%, 14.0 w%, 15.0 w%, 16.0 w%, 17.0 w%, 18.0 w%, 19.0 w%, 20,0 w%, 21.0 w%, 22.0 w%, 23.0 w%, 24.0 w%, 25.0 w%, 26.0 w%, 27.0 w%, 28.0 w%, 29.0 w%.

Preferably, the one or more additive of the dust suppressant liquid composition of the invention is chosen in the group comprising humectants, viscosity modifiers, preservatives, surfactants, dispersants, emulsifying agents, electrolytes, pH modifiers, pigments, deicers, and their combinations thereof.

Preferably, said humectant is chosen in the group comprising lignosulfonates, glycerin, sugar alcohols for example sorbitol, xylitol, molasses, vegetable oils and their combinations thereof.

Preferably, said preservative is 2-bromo-2-nitropropane-1,3-diol (bronopol) and the like.

Preferably, said surfactant is chosen in the group of Lutensol^{®} line of surfactants, Yucca schidigera extracts, ethoxylated alkyl phenols, organomodified silicones, rhamnolipids, sophorolipids, alcohol ethoxylates, and their combinations thereof.

Preferably, said deicer is an eco-friendly deicer, for example potassium formate and the like.

Preferably, the one or more additive of said dust suppressant liquid composition of the present invention is in the form of an aqueous phase.

Advantageously, the dust suppressant liquid composition of the present invention has a bulk density from 1.05 to 1.3 kg/L, for example 1.06 kg/L, 1.07 kg/L, 1.08 kg/L, 1.09 kg/L, 1.10 kg/L, 1.11 kg/L, 1.12 kg/L, 1.13 kg/L, 1.14 kg/L, 1.15kg/L, 1.16 kg/L, 1.17 kg/L, 1.18 kg/L, 1.19 kg/L, 1.20 kg/L, 1.21 kg/L, 1.22 kg/L, 1.23 kg/L, 1.24 kg/L, 1.25 kg/L, 1.26 kg/L, 1.27 kg/L, 1.28 kg/L, 1.29 kg/L.

The present invention also relates to a ready to use dust suppressant liquid composition comprising a weight ratio defined between said dust suppressant liquid composition according to the invention and water or between said dust suppressant solid formulation according to the invention and water, comprised from 99/1 to 1/99.

In addition, the incorporation of latex into ready to use dust suppressant liquid composition lies in its role as a food source and carbon supply for said series of dust suppressing bacteria species. As the ready to use dust suppressant liquid composition is applied to roads, the latex becomes readily available to the dust suppressing bacteria present in the solution. These dust suppressing bacteria thrive and proliferate on the treated surface.

As a result, the ready to use dust suppressant liquid composition becomes even more potent in its dust-controlling capabilities, while the bacteria flourish and continue their essential work in biofilm formation.

Other embodiments of the ready to use dust suppressant liquid composition according to the present invention are mentioned in the appended claims.

Advantageously, said weight ratio of the ready to use dust suppressant liquid composition of the invention defined between said dust suppressant liquid composition according to the invention and water or between said dust suppressant solid formulation according to the invention and water, is preferably from 95/5 to 5/95, from 90/10 to 10/90, from 85/15 to 15/85, from 80/20 to 20/80, from 75/25 to 25/75, from 70/30 to 30/70, from 65/35 to 35/65, from 60/40 to 40/60, from 55/45 to 45/50.

The present invention also relates to a method for suppressing dust, comprising applying the dust suppressant liquid composition according to the invention, or applying the ready to use dust suppressant liquid composition according to the invention, to a substrate, preferably the applying step is repeated more than once, more preferably twice, even more preferably three times, in particular four times, more particular five times, advantageously six times, more advantageously seven times, preferably eight times, in particular nine times, for example ten times.

Other embodiments of the method for suppressing dust according to the present invention are mentioned in the appended claims.

Advantageously, the substrate of the method of the present invention is chosen in the group comprising: a road, a surface comprising dust or dirt, a mining road, a construction site, trail path, racetrack, animal racing surface, horse track, stockpiles, dumping area, landfills, an unpaved surface.

The present invention also relates to the utilization of the dust suppressant liquid composition and/or the ready to use dust suppressant liquid composition according to the invention to suppress dust on a substrate, preferably on an unpaved surface.

Preferably, components and/or products and/or ingredients of the present invention are bio-based.

Other characteristics and advantages of the present invention will be derived from the non-limitative following description, and by making reference to the examples.

### Examples.-

Various formulations for dust suppressing were tested to evaluate the dust suppressing effect of the liquid composition of the present invention, by a Drop test.

### Example 1. - Samples preparation and dust suppression tests

Thirteen treatments were produced including one positive control (water only), one negative control (untreated), and eleven powder solid formulation (Table 1).

For dust suppressant tests, these solid formulations will further be combined with water and/or other liquid ingredients to produce liquid compositions.

**Table 1 :**

| | **Microbial fraction** | **Non-microbial fraction** | | |
|---|---|---|---|---|
| **Formula** | **Biofilm forming bacteria species** | **Latex binder** | **Xanthan Gum** | **Lignosulfonate** |
| F1 **(-)** | n/a | | | |
| F2 **(+)** | 2.0% | 98.0 % | | |
| F3 **(-)** | 100.0% | 0.0% | | |
| F4 **(-)** | 2.0% | 98.0 % | | |
| F5 **(-)** | | 100.0% | | |
| F6 **(-)** | | 100.0% | | |
| F7 **(-)** | 100.0% | | | |
| F8 **(**-**)** | n/a | | | |
| F9 **(+)** | 1.2 % | 58.2% | 40.7 % | |
| F10 **(+)** | 0.1 % | 3.5% | | 96.5% |
| F11 **(+)** | 0.1 % | 6.7 % | | 93.2% |
| F12 **(+)** | 0.8% | 41.4 % | 57.8% | |
| F13 **(+)** | 1.2 % | 58.2% | 40.7 % | |

| | | | | |
|---|---|---|---|---|
| **F1** condition **(-)** is water only (control). **F2** condition **(+)** is a dust suppressant solid formulation according to the invention including a solid mixture of 2.0% of a series of biofilm forming bacteria species and 98.0% of a latex binder. **F3** condition **(-)** is a comparative example of **F2** condition, including only bacteria (100%). This condition does not comprise a latex binder. **F4** condition **(-)** at the stage of solid formulation is identical to **F2** condition (2.0% of a series of biofilm forming bacteria species and 98.0% of a latex binder). **F5** condition **(-)** is a comparative example of **F2** condition, including only latex binder (100%). This condition does not comprise bacteria. **F6** condition **(-)** at the stage of solid formulation is identical to **F5** condition (100% latex binder). This condition does not comprise bacteria. **F7** condition **(-)** at the stage of solid formulation is identical to **F3** condition including only bacteria (100%). This condition does not comprise a latex binder. **F8** condition **(-)** is negative control (untreated stone). **F9** condition **(+)** is a dust suppressant solid formulation according to the invention including a mixture of 1.2% of said series of biofilm forming bacteria species, 58.2% of latex binder and 40.7% of xanthan gum. **F10** condition **(+)** is a dust suppressant solid formulation according to the invention including a mixture of 0.1% of said series of biofilm forming bacteria species, 3.5% of latex binder and 96.5% of lignosulfonate. **F11** condition **(+)** is a dust suppressant solid formulation according to the invention including a mixture of 0.1% of said series of biofilm forming bacteria species, 6.7% of latex binder and 93.2% of lignosulfonate. **F12** condition **(+)** is a dust suppressant solid formulation according to the invention including a mixture of 0.8% of said series of biofilm forming bacteria species, 41.4% of latex binder and 57.8% of xanthan gum. **F13** condition **(+)** is a dust suppressant solid formulation according to the invention including a mixture of 1.2% of said series of biofilm forming bacteria species, 58.2% of latex binder and 40.7% of xanthan gum. | | | | |

These formulations **F1** to **F13** have been combined with water and/or other liquid ingredients to produce liquid compositions **C1** to **C13** (Table 2).

**Table 2 :**

| **Liquid Compositions** | Solid formulation according to Table 1 | Glycerin | Water |
|---|---|---|---|
| C1 **(-)** | 0.00% | 0.00% | 100% |
| C2 **(+)** | 0.97% of F2 | 82.84% | 16.19% |
| C3 **(-)** | 0.02% of F3 | 82.84% | 17.14% |
| C4 **(-)** | 0.97% of F4 | | 99.03% |
| C5 **(-)** | 0.95% of F5 | 82.84% | 16.21% |
| C6 **(-)** | 0.95% of F6 | | 99.05% |
| C7 **(-)** | 0.02% of F7 | | 99.98% |
| C8 **(-)** | 0.00% | | |
| C9 **(+)** | 1.64% of F9 | 15.33% | 83.03% |
| C10 **(+)** | 27.64% of F10 | | 72.36% |
| C11 **(+)** | 14.31 % of F11 | 15.33% | 70.36% |
| C12 **(+)** | 2.31% of F12 | | 97.69% |
| C13 **(+)** | 1.64% of F13 | | 98.36% |

| | | | |
|---|---|---|---|
| **C1** condition **(-)** is water only applied to said untreated stone dust. **C2** condition **(+)** is a dust suppressant liquid composition according to the invention including a mixture of 0.97% of formulation **F2** with 82.84% glycerin and 16.19% of water. **C3** condition **(-)** is a comparative example of **C2** condition, including 0.02% of formulation **F3,** 82.84% glycerin and 17.14% of water. This composition **C3** does not comprise a latex binder. **C4** condition **(-)** is a comparative example of **C2** condition, including 0.97% of formulation **F4** and 99.03% of water. This composition **C4** does not comprise glycerin as an additive. **C5** condition **(-)** is a comparative example of **C2** condition, including 0.95% of formulation **F5,** 82.84% of glycerin and 16.21% of water. This composition **C5** does not comprise dust suppressing bacteria species. **C6** condition **(-)** is a comparative example of **C2** condition, including 0.95% of formulation **F6** and 99.05% of water. This composition **C6** does not comprise dust suppressing bacteria species nor additives. **C7** condition **(-)** is a comparative example of **C2** condition, including 0.02% of formulation **F7** and 99.98% of water. This composition **C7** does not comprise latex binder nor additives. **C8** condition **(-)** is negative control (untreated stone). **C9** condition **(+)** is a dust suppressant liquid composition according to the invention including a mixture of 1.64% of said formulation **F9** according to the invention with 15.33% glycerin and 83.03% of water. **C10** condition **(+)** is a dust suppressant liquid composition according to the invention including a mixture of 27.64% of said formulation **F10** according to the invention with 72.36% of water. **C11** condition **(+)** is a dust suppressant liquid composition according to the invention including a mixture of 14.31% of said formulation **F11** according to the invention with 15.33% glycerin and 70.36% of water. **C12** condition **(+)** is a dust suppressant liquid composition according to the invention including a mixture of 2.31 % of said formulation **F12** according to the invention with 97.69% of water. **C13** condition **(+)** is a dust suppressant liquid composition according to the invention including a mixture of 1.64% of said formulation **F13** according to the invention with 98.36% of water. | | | |

These liquid compositions C1 to C13 were diluted prior to application to create a ready-to-use composition by mixing each composition C1 to C13 at a rate of 75% C1-C13 to 25% water. These diluted ready-to-use compositions (RtU C) were then applied to 25-gram samples of untreated stone dust using a spraying nozzle to add 1 gram of solution, achieving an even distribution. Samples were stored in open containers until analysed for dust emissions, which took place on various date after treatment.

Dust emissions from each sample were evaluated by dropping **25** grams of material in a closed chamber while an attached dust monitor logged airborne particulate over time. The tests were performed **day 7** after treatment.

The Drop test methodology consists of:
1. Measure out 25 grams of each treated sample into clean labelled dishes.
2. Process each sample one at a time using the following drop test procedure:
   a. Connect the DustTrak DRX Aerosol Monitor 8533 to the drop cylinder using the flexible hose (Figure 3).
   b. Set the DustTrak RunMode to 2-minute duration, with 1 second sampling frequency.
   c. Start the DustTrak logging.
   d. Wait 30 seconds to allow readings to settle to a consistent background level.
   e. Lift the top cover of the cylinder, quickly drop the sample material into the cylinder and replace the top cover.
   f. Allow the DustTrack to run for the full 2-minute duration.
   g. Disconnect the DustTrack, clean the drop cylinder using cloths and anti-static sheets.
   h. Discard the dropped sample material and clean the bottom plate.
   i. Replace equipment and proceed to the next sample.
3. Import the data into Excel, calculate the maximum dust level recorded for each sample and produce summary statistics.

Results Table 3 :

| ***Ready-to-use composition*** | ***Maximum PM10 (mg*/*****m3)** 7 days* after treatment *Average* of 3 samples per treatment | |
|---|---|---|
| | **Average** | **% difference v. Control (C1)** |
| Ready to use C1 **(-)** | 142.67 | n/a |
| Ready to use C2 **(+)** | 32.28 | **-77%** |
| Ready to use C3 **(-)** | 55.03 | -61% |
| Ready to use C4 **(-)** | 131.93 | -8% |
| Ready to use C5 **(-)** | 21.69 | -85% |
| Ready to use C6 **(-)** | 103.63 | -27% |
| Ready to use C7 **(-)** | 123.87 | -13% |
| Ready to use C8 **(-)** | 150 | +5% |
| Ready to use C9 **(+)** | 114.4 | **-20%** |
| Ready to use C10 **(+)** | 29.17 | **-80%** |
| Ready to use C11 **(+)** | 43 | **-70%** |
| Ready to use C12 **(+)** | 97.23 | **-32%** |
| Ready to use C13 **(+)** | 62.4 | **-56%** |

### Discussion of results :

As previously explained the dust suppressant liquid composition of the present invention and the ready-to-use composition of the present invention present a triple action when applied to the substrate to be treated.

Firstly, the water will act immediately as humidifying agent by weighing down the dust particles and reducing their ability to become airborne.

This first action takes place during the first 1 to 5 days of application and explains why after 7 days the ready-to-use C1 (-) condition which is water only gives results similar to the untreated stone (Ready-to-use C8 (-) condition), due to evaporation of water.

In parallel, the additives (glycerin and/or xanthan gum) and the polymer (latex binder and/or lignosulfonate) together with the aqueous phase will act both as creating a flexible and resilient surface avoiding airborne particles and as protective matrix for the growing of the bacteria to initiate the formation of the biofilm.

This second action starts in parallel with the water action and continues to act after 7 days from the application.

Thirdly and again in parallel of the first and second actions, due to the presence of the latex binder and/or the lignosulfonate and/or the glycerin and/or the xanthan gum as a food source and carbon supply for said series of dust suppressing bacteria species, the bacteria actively generate a biofilm on the stone surface enhancing their ability to adhere firmly and ensuring maximum coverage and prolonged activity to suppress dust emissions.

This third action starts slowly the first 7 days after application and shows a significant effect after 7 days after application.

The ready-to-use C2 composition according to the present invention shows a reduction of -77% of PM10 (mg/m3) of dust in comparison to the control condition (water only).

In comparison to comparative examples (ready-to-use C3 and C4 conditions) with respectively no latex binder and no glycerin show inferior results in terms of dust reduction, respectively -61 % and -8%.

Also in comparison, ready-to-use C5 condition without bacteria shows a reduction of -85% of dust after 7 days which is quite similar to the ready-to-use C2 composition, explained by the fact that at 7 days the bacteria have not yet shown a significant effect, and because there is 2% more latex binder.

Ready-to-use C6 and C7 compositions, respectively polymer only and bacteria only, show inferior results in terms of dust reduction, respectively -27% and -13% in comparison to the ready-to-use C2 composition.

Finally, ready-to-use composition C9, C10, C11, C12 and C13 according to the present invention show a reduction respectively of -20%, -80%, -70%, -32% and -56% of PM10 (mg/m3) of dust in comparison to the control condition (water only). These results are promising as alternatives to the utilization of glycerin with similar effects.

Indeed, the ready-to-use compositions C9 to C13 of the present invention using latex binder and/or lignosulfonate and/or xanthan gum instead of glycerin provided significant dust control versus water condition.

### Example 2. - Microbial effect over time

Dust emissions from each Ready-to-use compositions C1 (-), C2 (+) and C5 (-) were evaluated by dropping **25** grams of material in a closed chamber while an attached dust monitor logged airborne particulate over time. The tests were performed **1, 3, 7, 10, 14, and 21 days** after treatment.

**Table 4 :**

| | ***Average maximum PM10 (mg*/*m3)* - *(2 samples)*** | | | | | |
|---|---|---|---|---|---|---|
| | **Day 1** | **Day 3** | **Day 7** | **Day 10** | **Day 14** | **Day 21** |
| Ready-to-use C1 **(-)** | 138.00 | 130.00 | 148.50 | 150.00 | 150.00 | 150 |
| Ready-to-use C2 **(+)** | 10.09 | 1.29 | 1.22 | 12.58 | 2.10 | 2.98 |
| Ready-to-use C5 **(-)** | 0.24 | 0.40 | 1.37 | 5.44 | 18.15 | 24.5 |

| | | | | | | |
|---|---|---|---|---|---|---|
| **Ready-to-use C1** condition **(-)** is water only applied to said untreated stone dust. **Ready-to-use C2** condition **(+)** is 75% of [a dust suppressant liquid composition according to the invention including a mixture of 0.97% of formulation **F2** with 82.84% glycerin and 16.19% of water] diluted with 25% of water. **Ready-to-use C5** condition **(-)** is a comparative example of **C2** condition, including 75% of [0.95% of formulation **F5,** 82.84% of glycerin and 16.21 % of water] diluted with 25% of water. This **ready-to-use C5** does not comprise dust suppressing bacteria species. | | | | | | |

### Discussion of the results:

The ready-to-use C2 (+) composition according to the present invention, in comparison to the water condition C1 (-) (control) and the ready-to-use C5 (-) composition which does not comprise biofilm forming bacteria, shows that due to the presence of the latex binder as a food source and carbon supply for said series of dust suppressing bacteria species, the bacteria actively generate a biofilm on the stone surface enhancing their ability to adhere firmly and ensuring maximum coverage and prolonged activity to suppress dust emissions.

Indeed, as it appears from Table 4, in the Ready-to-use C2 condition of the invention, the loss of activity of the first action of the water and the second action of the additives (glycerin) and the polymer (latex binder) together with the aqueous phase, is compensated from the 7th day by the development of the biofilm thanks to the presence of bacteria, this being even more visible on the 14th and 21 st days of the test.

In comparison, the ready-to-use C5 composition, which does not comprise dust suppressing bacteria species, shows that the effect for Day 1, Day 3 and Day 5 is quite similar to ready-to-use C2 composition of the invention. However, after 10 days, especially tests done at day 14 and day 21, it clearly appears that the effect of the water and additives and polymer is decreasing, and due to the lack of biofilm forming bacteria the amount of dust emission is significantly increasing.

It should be understood that the present invention is not limited to the described embodiments and that variations can be applied without going outside of the scope of the appended claims.

## Claims

1. Dust suppressant solid formulation to be diluted in an aqueous phase, comprising a first microbial fraction comprising a series of biofilm forming bacteria species and a second non-microbial fraction, comprising at least latex binder.

2. Dust suppressant solid formulation according to claim 1, wherein said second non-microbial fraction comprises a further binder chosen from the group comprising lignosulfonate, xanthan gum, acacia gum, cellulose and the like, their derivates and their combination.

3. Dust suppressant solid formulation according to claim 1 or 2, wherein said latex binder is a biobased latex binder, in particular a biobased latex binder having surfactant properties, more preferably a biobased starch latex binder, more preferably a biobased modified starch latex binder.

4. Dust suppressant solid formulation according to any of claim 1 to 3, wherein the dust suppressant solid formulation comprises from 95 to 99.9 weight% of the second non-microbial fraction with respect to the total weight of the dust suppressant solid formulation and from 0.1 to 5 weight% of the first microbial fraction with respect to the total weight of the dust suppressant solid formulation, preferably comprising from 95 to 99.5 weight% of said second non-microbial fraction and from 0.5 to 5 weight % of said microbial fraction, advantageously comprising from 96 to 99 weight% of said second non-microbial fraction and from 1 to 4 weight% of said first microbial fraction, more preferably comprising from 96 to 98 weight% of said second non-microbial fraction and from 2 to 4 weight% of said first microbial fraction, even more preferably comprising from 97 to 98 weight% of said second non-microbial fraction and from 2 to 3 weight% of said first microbial fraction .

5. Dust suppressant solid formulation according to any of the preceding claims, wherein said second non-microbial fraction is in the form of a powder.

6. Dust suppressant solid formulation according to any of the claims 1 to 5, wherein said series of biofilm forming bacteria species comprises one or more biofilm forming bacteria species chosen in the group comprising Achromobacter, Acetonema, Actinobacter, Alcaligenes, Alkalibacillus, Ammoniphilus, Amphibacillus, Anaerobacter, Anaerospora, Aneurinibacillus, Anoxybacillus, Arthrobacter, Bacillus, Brevibacillus, Caldanaerobacter, Caloramator, Caminicella, Cerasibacillus, Clostridium, Clostridiisalibacter, Cohnella, Dendrosporobacter, Desulfotomaculum, Desulfosporomusa, Desulfosporosinus, Desulfovirgula, Desulfunispora, Desulfurispora, Enterobacter, Filifactor, Filobacillus, Flavobacterium, Gelria, Geobacillus, Geosporobacter, Gracilibacillus, Halonatronum, Heliobacterium, Heliophilum, Laceyella, Lentibacillus, Lysinibacillus, Mahella, Metabacterium, Moorella, Natroniella, Oceanobacillus, Orenia, Ornithinibacillus, Oxalophagus, Oxobacter, Paenibacillus, Paraliobacillus, Pelospora, Pelotomaculum, Piscibacillus, Planifilum, Pontibacillus, Pseudomonas, Propionispora, Rhodococcus, Salinibacillus, Salsuginibacillus, Seinonella, Shimazuella, Sporacetigenium, Sporoanaerobacter, Sporobacter, Sporobacterium, Sporohalobacter, Sporolactobacillus, Sporomusa, Sporosarcina, Sporotalea, Sporotomaculum, Syntrophomonas, Syntrophospora, Tenuibacillus, Tepidibacter, Terribacillus, Thalassobacillus, Thermoacetogenium, Thermoactinomyces, Thermoalkalibacillus, Thermoanaerobacter, Thermoanaeromonas, Thermobacillus, Thermoflavimicrobium, Thermovenabulum, Tuberibacillus, Virgibacillus, Vulcanobacillus, preferably comprising two or more biofilm forming bacteria species, more preferably comprising three or more biofilm forming bacteria species, more preferably comprising four or more biofilm forming bacteria species, even more preferably comprising five or more biofilm forming bacteria species.

7. Dust suppressant solid formulation according to claim 1 to 6, wherein said series of biofilm forming bacteria species comprises one or more biofilm forming bacteria species chosen in the group comprising Bacillus amyloliquefaciens, Bacillus licheniformis, Bacillus megaterium, Bacillus pumilis, Bacillus subtilis, Bacillus subtilis subspecies inaquosorum, Bacillus subtilis subspecies subtilis, Bacillus velezensis, preferably comprising two or more biofilm forming bacteria species, more preferably comprising three or more biofilm forming bacteria species, more preferably comprising four or more biofilm forming bacteria species, even more preferably comprising five or more biofilm forming bacteria species.

8. Dust suppressant solid formulation according to claims 1 to 7, wherein said series of biofilm forming bacteria species contains:
- at least 0.1 million (1×10⁵), in particular at least 0.5 million (5×10⁵), more particular at least 0.8 million (8×10⁵), preferably at least 1 million (1×10⁶), more preferably at least 2 million (2×10⁶), in particular at least 4 million (4×10⁶), particularly at least 4.7 million (4.7×10⁶), preferably at least 5 million (5×10⁶), more preferably at least 10 million (1×10⁷), in particular at least 20 million (2×10⁷), particularly at least 50 million (5×10⁷) preferably at least 70 million (7×10⁷), more preferably at least 100 million (1×10⁸), particularly at least 150 million (1.5×10⁸) preferably at least 200 million (2×10⁸), more preferably at least 250 million (2.5×10⁸), in particular at least 300 million (3×10⁸), particularly at least 350 million (3.5×10⁸), more preferably at least 400 million (4×10⁸), particularly at least 450 million (4.5×10⁸), more preferably at least 500 million (5×10⁸), cfu of Bacillus licheniformis per gram of the dust suppressant solid formulation measured according to ASTM-D5465-93 (dated 1998) standard, and/or
- at least 10 million (1×10⁷), preferably at least 50 million (5×10⁷), more preferably at least 100 million (1×10⁸), in particular at least 200 million (2×10⁸), particularly at least 300 million (3×10⁸), advantageously at least 400 million (4×10⁸), more particular at least 500 million (6×10⁸), in particular at least 700 million (7×10⁸), preferably at least 800 million (8×10⁸), even more preferably at least 900 million (9×10⁸), for example at least 920 million (9.2×10⁸), preferably at least 950 million (9.5×10⁸), more preferably at least 1 billion (1×10⁹), cfu of Bacillus megaterium per gram of the dust suppressant solid formulation measured according to ASTM-D5465-93 (dated 1998) standard, and/or
- at least 100 thousand (1×10⁵), preferably at least 300 thousand (3×10⁵), more particular 500 thousand (5×10⁵), more preferably at least 830 thousand (8.3×10⁵), in particular at least 1 million (1×10⁶), particularly at least 2 million (2×10⁶), advantageously at least 3 million (3×10⁶), more particular at least 4 million (4×10⁶), cfu of Bacillus amyloliquefaciens per gram of the dust suppressant solid formulation measured according to ASTM-D5465-93 (dated 1998) standard, and/or
- at least 100 million (1×10⁸), particularly at least 250 million (2.5×10⁸) preferably at least 400 million (4×10⁸), more preferably at least 600 million (6×10⁸), in particular at least 750 million (7.5×10⁸), particularly at least 800 million (8×10⁸), more preferably at least 900 million (9×10⁸), in particular at least 1 billion (1×10⁹), preferably at least 2 billion (2×10⁹), more particular 2.8 billion (2.8×10⁹), more preferably at least 3.6 billion (3.6×10⁹), in particular at least 5 billion (5×10⁹), particularly at least 7 billion (7×10⁹), advantageously at least 7.2 billion (7.2×10⁹), more particular at least 8 billion (8×10⁹), particularly at least 8.5 billion (8.5×10⁹), cfu of Bacillus subtilis per gram of the dust suppressant solid formulation measured according to ASTM-D5465-93 (dated 1998) standard, and/or
- at least 0.1 billion (1×10⁸), preferably at least 0.3 billion (3×10⁸), more particular 0.5 billion (5×10⁸), more preferably at least 0.8 billion (8×10⁸), in particular at least 1 billion (1×10⁹), particularly at least 2 billion (2×10⁹), advantageously at least 3 billion (3×10⁹), more particular at least 3.2 billion (3.2x10⁹), preferably at least 4 billion (4×10⁹), advantageously at least 5 billion (5×10⁹), more particular at least 7 billion (7×10⁹) cfu of Bacillus subtilis subspecies Inaquosorum per gram of the dust suppressant solid formulation measured according to ASTM-D5465-93 (dated 1998) standard, and/or
- at least 0.1 billion (1×10⁸), preferably at least 0.3 billion (3×10⁸), more particular 0.5 billion (5×10⁸), more preferably at least 0.8 billion (8×10⁸), in particular at least 1 billion (1×10⁹), particularly at least 2 billion (2×10⁹), advantageously at least 3 billion (3×10⁹), more particular at least 3.2 billion (3.2x10⁹), preferably at least 4 billion (4×10⁹), advantageously at least 5 billion (5×10⁹), more particular at least 7 billion (7×10⁹) cfu of Bacillus subtilis subspecies subtilis per gram of the dust suppressant solid formulation measured according to ASTM-D5465-93 (dated 1998) standard, and/or
- at least 10 million (1×10⁷), preferably at least 50 million (5×10⁷), more particular 100 million (1×1 0⁸), more preferably at least 200 million (2×10⁸), in particular at least 300 million (3×10⁸), particularly at least 350 million (3.5×10⁸), advantageously at least 400 million (4×10⁸), more particular at least 450 million (4.5×10⁸), even more preferably at least 500 million (5×10⁸) cfu of Bacillus pumilis per gram of the dust suppressant solid formulation measured according to ASTM-D5465-93 (dated 1998) standard, and/or
- at least 0.1 billion (1×10⁸), preferably at least 0.5 billion (5×10⁸), more particular 0.8 billion (8×10⁸), more preferably at least 1 billion (1×1 0⁹), in particular at least 2 billion (2×10⁹), particularly at least 2.5 billion (2.5×10⁹), advantageously at least 3 billion (3×10⁹), more particular at least 3.5 billion (3.5×10⁹), even more preferably at least 4 billion (4×10⁹), advantageously at least 4.5 billion (4.5×10⁹), in particular at least 5 billion (5×10⁹) cfu of Bacillus velezensis per gram of the dust suppressant solid formulation measured according to ASTM-D5465-93 (dated 1998) standard.

9. Dust suppressant solid formulation according to any of the preceding claims, wherein said second non-microbial fraction of said dust suppressant solid composition comprises one or more solid additive that is chosen in the group comprising humectants, viscosity modifiers, preservatives, surfactants, dispersants, electrolytes, emulsifying agents, pH modifiers, pigments, deicers, and their combinations thereof.

10. Dust suppressant liquid composition comprising:
- from 10 to 99 weight% of water based on the weight of said liquid composition, preferably from 15 w% to 99 w%, in particular from 20 w% to 99w%, more particular from 25 w% to 99 w%, even more particular 30 w% to 99 w%, advantageously from 35 w% to 99w% preferably from 40 w% to 99 w%, more preferably from 50 w% to 99 w%, even more preferably from 60 w% to 99 w%, advantageously from 70 w% to 99 w%, in particular from 75 w% to 99 w%,
- from 10 to 90 weight% of one or more additive based on the weight of said liquid composition, preferably from 10 w% to 85 w%, preferably from 10 w% to 80 w%, more preferably from 10 w% to 70 w%, even more preferably from 10 w% to 60 w%, advantageously from 10 w% to 50 w%, in particular from 10 w% to 40 w%, particularly from 10 w% to 30 w%, even more preferably from 10 w% to 25 w%, advantageously from 15 w% to 20 w%,
- from 0.1 to 30 weight% of the dust suppressant solid formulation according to any one of claim 1 to 9 based on the weight of said liquid composition, preferably from 0.1 w% to 30 w%, for example 0.5 w%, 1 w%, 1.5 w%, 2.0 w%, 3.0 w%, 4.0 w%, 5.0w%, 6.0 w%, 7.0 w%, 8.0 w%, 9.0 w%, 10.0 w%, 11.0 w%, 12.0 w%, 13.0 w%, 14.0 w%, 15.0 w%, 16.0 w%, 17.0 w%, 18.0 w%, 19.0 w%, 20,0 w%, 21.0 w%, 22.0 w%, 23.0 w%, 24.0 w%, 25.0 w%, 26.0 w%, 27.0 w%, 28.0 w%, 29.0 w%.

11. Dust suppressant liquid composition according to claim 10, wherein said one or more additive is chosen in the group comprising humectants, viscosity modifiers, preservatives, surfactants, dispersants, emulsifying agents, electrolytes, pH modifiers, pigments, deicers, and their combinations thereof.

12. Dust suppressant liquid composition according to one of claims 10 to 11, having a bulk density from 1.05 to 1.3 kg/L, for example 1.06 kg/L, 1.07 kg/L, 1.08 kg/L, 1.09 kg/L, 1.10 kg/L, 1.11 kg/L, 1.12 kg/L, 1.13 kg/L, 1.14 kg/L, 1.15kg/L, 1.16 kg/L, 1.17 kg/L, 1.18 kg/L, 1.19 kg/L, 1.20 kg/L, 1.21 kg/L, 1.22 kg/L, 1.23 kg/L, 1.24 kg/L, 1.25 kg/L, 1.26 kg/L, 1.27 kg/L, 1.28 kg/L, 1.29 kg/L.

13. Ready to use dust suppressant liquid composition comprising a weight ratio defined between said dust suppressant liquid composition according to one of claims 10 to 12 and water or between said dust suppressant solid formulation according to one of claims 1 to 9 and water, comprised from 99/1 to 1/99, preferably from 95/5 to 5/95, from 90/10 to 10/90, from 85/15 to 15/85, from 80/20 to 20/80, from 75/25 to 25/75, from 70/30 to 30/70, from 65/35 to 35/65, from 60/40 to 40/60, from 55/45 to 45/50.

14. Method for suppressing dust, comprising applying the dust suppressant liquid composition as defined in any one of claims 10 to 12, or applying the ready to use dust suppressant liquid composition according to claim 13, to a substrate, preferably the applying step is repeated more than once, more preferably twice, even more preferably three times, in particular four times, more particular five times, advantageously six times, more advantageously seven times, preferably eight times, in particular nine times, for example ten times.

15. Method for suppressing dust according to claim 14 wherein said substrate is chosen in the group comprising: a road, a surface comprising dust or dirt, a mining road, a construction site, trail path, racetrack, animal racing surface, horse track, stockpiles, dumping area, landfills, an unpaved surface.

16. Utilization of the dust suppressant liquid composition according to any one of claims 10 to 12 and/or the ready to use dust suppressant liquid composition as defined in claims 13, to suppress dust on a substrate, preferably on an unpaved surface.
